(19) Europäisches Patentamt
European Patent Office
Office européen des brevets

(11) Veröffentlichungsnummer: **0 070 566**
**B1**

# (12) EUROPÄISCHE PATENTSCHRIFT

(45) Veröffentlichungstag der Patentschrift: **10.10.84**

(51) Int. Cl.³: **C 07 C 35/31**, C 11 B 9/00, A 61 K 7/46, C 07 C 29/40

(21) Anmeldenummer: **82106549.7**

(22) Anmeldetag: **20.07.82**

(54) **C-8-substituierte 1,5-Dimethyl-bicyclo (3.2.1)octan-8-ole.**

(30) Priorität: **21.07.81 DE 3128790**

(43) Veröffentlichungstag der Anmeldung: **26.01.83 Patentblatt 83/4**

(45) Bekanntmachung des Hinweises auf die Patenterteilung: **10.10.84 Patentblatt 84/41**

(84) Benannte Vertragsstaaten: **CH FR GB LI NL**

(56) Entgegenhaltungen:
**FR - A - 2 441 604**
**GB - A - 2 020 659**

(73) Patentinhaber: **Dragoco Gerberding & Co. GmbH, Dragocostrasse 1, D-3450 Holzminden (DE)**

(72) Erfinder: **Brunke, Ernst-Joachim, Dr., Holbeinstrasse 6, D-3450 Holzminden (DE)**
Erfinder: **Struwe, Hartmut, Kokenhammer 23, D-3450 Holzminden (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

EP 0 070 566 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue an C-Atom 8 substituierte 1,5-Dimethyl-bicyclo[3.2.1]octan-8-ole der allgemeinen Formel I, worin R eine niedere geradkettige oder verzweigte Alkyl-, Alkenyl- oder Alkinylgruppe mit bis zu 6 C-Atomen darstellt, und die geschlängelte Linie an C-Atom 8 die epimeren Formen bedeutet.

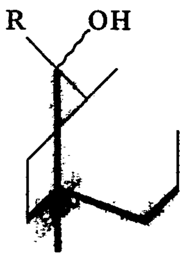

I

bzw.

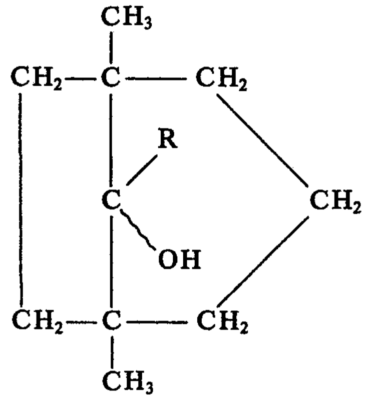

Die Erfindung betrifft zudem das Verfahren zur Herstellung der Verbindungen der allgemeinen Formel I sowie ihre Verwendung als Riechstoffe und somit auch Parfümkompositionen, die durch einen Gehalt an einer Verbindung der allgemeinen Formel I gekennzeichnet sind.

Es wurde gefunden, daß die neuen an C-8 substituierten 1,5-Dimethyl-bicyclo[3.2.1]octan-8-ole der allgemeinen Formel I wertvolle stabile Riechstoffe sind. Die Verbindungen der allgemeinen Formel I besitzen erdige, holzige und frische Geruchsnoten, die teilweise eine extrem starke Geruchsintensität aufweisen.

In der Parfümerie wird seit langem Patchouli-Öl eingesetzt. Dieses ätherische Öl von Pogostemon patchouly besitzt einen ausgeprägt holzig-erdigen Duftkomplex und einen ausstrahlenden Effekt, der sich vor allem in Parfümkompositionen entfaltet. Als olfaktorisch bedeutungsvollste Inhaltsstoffe des Patchouli-Öls sind Patchoulol (A) und Norpatchoulenol (B) beschrieben worden. Für A und B sind keine industriell verwendbaren Synthesen bekannt.

Das durch Mikroorganismen erzeugte Geosmin (C) bewirkt den Geruch frischer Erde. Wie anhand von synthetisiertem Geosmin gefunden wurde, besitzt diese Substanz eine der höchsten bisher gefundenen Geruchsintensitäten. In dem US-Patent 4 248 742 ist die Verwendung eines Gemisches von Geosmin-Stereoisomeren in Parfum-Kompositionen beschrieben worden. Dieses Isomeren-Gemisch wurde nach Vorschriften von Marshall et al. (J. Org. Chem. 33, 2593 [1969]) und Ayer et al. (Canad. J. Chem. 54, 3276 [1976]) dargestellt. Diese Vorschriften werden jedoch nicht industriell ausgeführt.

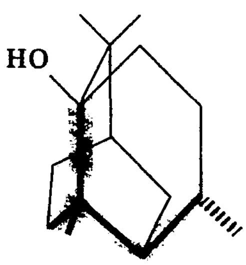

A

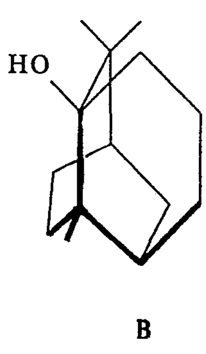

B

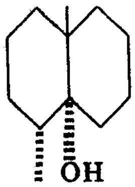

C

Die zeitweise begrenzte Verfügbarkeit des Patchouli-Öls und dessen sehr unterschiedliche Geruchsqualität und die Nichtverfügbarkeit des Geosmins sind Veranlassung, synthetische Riechstoffe zu entwickeln, die einen oder mehrere ölfaktorische Aspekte des Patchouli-Öls, bzw. den des Geosmins ergeben. Derartige Riechstoffe sollten in notwendigen Mengen mit konstanter Geruchsqualität hergestellt werden können.

Bisher stehen eine Reihe von einheitlichen Riechstoffen zur Verfügung, deren Geruch aus einer Kombination von erdigen mit camphrigen Aspekten besteht. Riechstoffe mit ausgeprägt camphrigen Geruchsnoten können nur sehr begrenzt in der Parfümerie verwendet werden und sind beispielsweise nicht als Patchouliöl-Substitut geeignet.

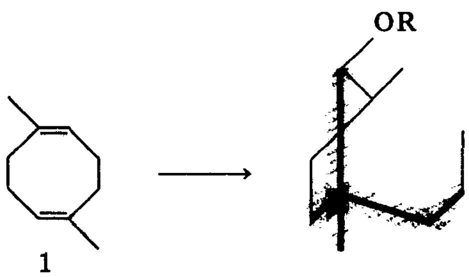

1

2: R = H

3: R = 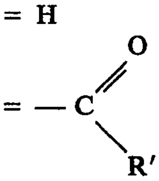

Der sekundäre Alkohol 2 mit 1,5-Dimethyl-bicyclo[3.2.1]octan-Grundgerüst, dessen Darstellung aus 1,5-Dimethyl-1,5-cyclo-octadien (1) I. K. Whitesell et al. (Tetrahedron Letters, 1976, S. 1549—1552) und A. I. Mulder und A. I. de Jong (UK-Patent Application 2020659A) beschrieben haben, besitzt einen unangenehmen technisch-camphrigen Geruch. In der UK-Patent Appl. 2020659A werden die Ester 3 (R' = Me, Et, Pr, . . .) des Alkohols 2 als Riechstoff beansprucht, da sie im Gegensatz zu 2 angenehm blumige, würzige und holzige Geruchsnoten besitzen.

2 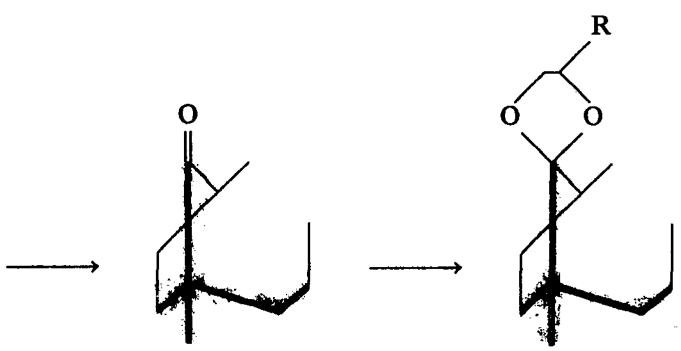

4 D

Die DE-OS 2 945 812 beschreibt die Oxidation von 2, die das Keton 4, ergibt. 4 besitzt gemäß dieser Offenlegungsschrift einen Geruch nach Koniferen und Campher mit einer würzigen Note. In der gleichen DE-OS sind die aus 4 dargestellten Acetale D beschrieben, die campherartige sowie eucalyptus-, rosmarin- und rosenähnliche Geruchsnoten aufweisen. Das Ethylenglykolacetal hat einen borneolartigen Geruch mit einer schwach erdigen Nebennote (also bei R = H).

Es wurde überraschenderweise festgestellt, daß die neuartigen, aus 4 dargestellten tertiären Alkohole der allgemeinen Formel I einen natürlich wirkenden erdigen Geruch mit sehr großer olfaktorischer Intensität aufweisen. Bei einigen Derivaten treten zusätzlich holzige Nebennoten auf, die in der Parfümerie durchaus erwünscht sind. Nur bei einigen Verbindungen ist ein typisch camphriger Geruch festzustellen. Die erfindungsgemäßen Verbindungen der allgemeinen Formel I sind daher als Riechstoff verwendbar und lassen sich in Parfümkompositionen äußerst wirkungsvoll einsetzen. Die Stabilität der Verbindungen der allgemeinen Formel I erlaubt deren Verwendung zur Parfümierung chemisch aggressiver Medien.

Die erdigen Geruchsnoten der Verbindungen der allgemeinen Formel I kann vorteilhaft auch für die Nacharbeitung ätherischer Öle oder Aromenkonzentrate verwendet werden, denen sie selbst in sehr kleinen Dosierungen Natürlichkeit verleihen.

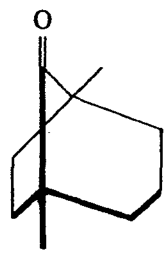

4

Na/NH$_3$ | C$_2$H$_2$

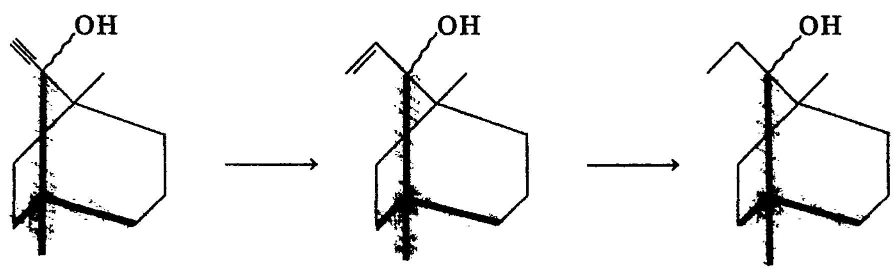

5 6 7

RX/Li

4

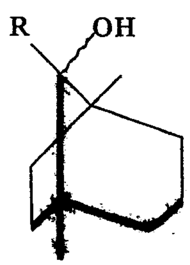

7: R = Ethyl
8: R = Methyl
9: R = n-Propyl
10: R = i-Propyl

11: R = n-Butyl
12: R = 1-Methyl-propyl
13: R = 2-Methyl-propyl
14: R = Allyl
15: R = n-Hexyl
16: R = 3-Methyl-butyl
17: R = Cyclopentyl
18: R = Cyclohexyl

Die Herstellung der Verbindungen der allgemeinen Formel I erfolgt in an sich bekannter Weise durch Umsetzen von 1,5-Dimethyl-bicyclo[3.2.1]octan-8-on (3) mit metallorganischen Verbindungen. So ergibt die Anlagerung von metallierten Acetylen unter vorzugsweiser Verwendung von Kaliumhydroxid in n-Butanol/Dimethylformamid das Ethinylcarbinol 5, das durch selektive Hydrierung unter vorzugsweiser Verwendung von Lindlar-Katalysator zu dem Vinylcarbinol 6, bzw. durch vollständige Hydrierung unter vorzugsweiser Verwendung von Raney-Nickel zu dem Ethyl-Derivat 7 führt. Außer diesen vorzugsweise verwendeten Reaktionsbedingungen sind weitere chemische Methoden und Verfahren, die zu den erfindungsgemäßen Substanzen 5, 6 und 7 führen, allgemein bekannt. So kann die Addition von Acetylen auch unter Verwendung von Natrium- oder Lithiumamid in flüssigem Ammoniak, und die Hydrierung zu 7 unter Verwendung verschiedener Edelmetall-Katalysatoren ausgeführt werden.

Die Carbinole 8—18 wurden vorzugsweise durch Umsetzen von 4 mit den entsprechenden lithiumorganischen Verbindungen (R—Li) und anschließende Hydrolyse erhalten. Bevorzugte Lösungsmittel waren hierbei Diethylether oder Tetrahydrofuran. Die Reaktionstemperaturen lagen vorzugsweise zwischen Raum- und Siedetemperatur und die Reaktionszeiten bei 0,3 molaren Ansätzen zwischen 1 und 3 Stunden. Bei der Durchführung von Grignard-Reaktionen mit dem Keton 4 entstanden neben den gewünschten tertiären Carbinolen 7—18 stets auch größere Mengen des sekundären Alkohols 2 als Produkt einer Grignard-Reduktion.

Tabelle 1

Massenspektroskopische Daten der Verbindungen der allgemeinen Formel I

| R | % bezogen auf Basispeak $M^{+\cdot}$ | $[M-18]^{+\cdot}$ | $[M-R]^{+\cdot}$ m/z 153 | $[M-(R+18)]^{+\cdot}$ m/z 135 | $R^{+\cdot}$ |
|---|---|---|---|---|---|
| methyl- | 33 | 27 | 28 | 46 | nicht registriert |
| ethyl- | 2 | 2 | 100 | 56 | 19 |
| n-propyl- | 2 | 2 | 100 | 67 | 50 |
| isopropyl- | 3 | — | 50 | 54 | 100 |
| n-butyl- | 2 | 1 | 100 | 61 | 20 |
| isobutyl- | 6 | 2 | 100 | 72 | 38 |
| sec.-butyl- | 6 | — | 61 | 77 | 51 |
| isoamyl- | 2 | 1 | 100 | 47 | 12 |
| cyclopentyl- | 6 | 1 | 61 | 48 | 100 |
| hexyl- | 2 | 1 | 100 | 44 | 6 |
| cyclohexyl- | 3 | — | 100 | 59 | 50 |
| vinyl- | 5 | 3 | 1 | 3 | 46 |
| ethinyl- | 1 | 1 | 1 | 100 | nicht registriert |

Die Strukturen der neuen Verbindungen der Formel I werden spektroskopisch charakterisiert durch Infrarot-, $^1$H-Kernresonanz- sowie Massenspektren (siehe auch die Herstellungsbeispiele 1 bis 7). Massenspektrometrisch nachweisbar ist der relativ kleine Molpeak sowie der durch Wasserabspaltung entstehende $[M-18]^+$-Peak. Signifikant für die Verbindung mit gesättigtem Rest an C-Atom 8 ist das durch Abspaltung dieses Restes zustande kommende Bruchstück mit m/z = 153. Das aus dem Rest entstandene Fragmention $R^+$. ist (bis auf 7) stets nachweisbar. Ebenfalls ausgeprägt ist das durch Abspaltung von Wasser und den C-8-Resten entstehende Fragmention m/z = 135 (siehe Tabelle 1). Die Konstitution der neuen Verbindungen wird durch das $^1$H-NMR-Signal bei 0,90 ppm (6H) für die beiden Brückenkopf-Methylgruppen charakterisiert.

Tabelle 2

Geruchsbeschreibung der Carbinole 5—18 (1%,in Ethanol)

| Verbindung | Geruchsbewertung nach 5 min | Geruchsbewertung nach 2 h |
|---|---|---|
| 5 | (sehr intensiv) angenehm erdig, weich, natürlich frisch | geruchlos |
| 6 | (sehr intensiv) frisch erdig, etwas camphrig | geruchlos |
| 7 | (extrem intensiv) natürlich, frisch erdig | geruchlos |
| 8 | (extrem intensiv) erdig, schimmlig | geruchlos |
| 9 | (mittlere Intensität) betont erdig-camphrig, leicht krautig, frisch | schwach holzig |
| 10 | (sehr intensiv) erdig, etwas holzig | holzig-erdig |
| 11 | (relativ schwach) betont erdig-camphrig (ähnlich 9, aber etwas schwächer) | leicht holzig |
| 12 | (relativ schwach) erdig-holzig | sehr schwach holzig |
| 13 | (mittlere Intensität) erdig, leicht holzig (ähnlich 10, jedoch etwas stärker holzig) | leicht holzig |
| 14 | (sehr intensiv) typisch camphrig, erdig | deutlich im selben Typ erhalten |
| 15 | sehr schwach | fast geruchlos |
| 16 | (mittlere Intensität) Labdanum-Note, ledrig | schwach holzig |
| 17 | (mittlere Intensität) erdig, ausgeprägt holzig (ähnlich 10) | holzig |
| 18 | (relativ schwach) leicht camphrig-holzig | sehr schwach holzig |

Die Geruchseigenschaften der Verbindungen 5 bis 18 wurden von einem Experten-Team ermittelt (siehe Tabelle 2). Obwohl sämtliche der genannten Verbindungen bemerkenswerte geruchliche Eigenschaften aufweisen, die eine industrielle Nutzung ermöglichen können, sind als besonders interessante Riechstoffe die Verbindungen der Formel I mit R = Methyl, Ethyl, iso-Propyl, Vinyl oder Ethinyl hervorzuheben. Diese Substanzen haben eine erhebliche Geruchsintensität und bewirken bei der Einarbeitung in Parfüm-Öle, selbst bei sehr niedriger Dosierung, deutliche und sehr gewünschte Effekte. Dies wird in den Beispielen 8 und 9 gezeigt.

Aus den Alkoholen 5 bis 18 wurden durch übliche Veresterungsreaktionen die zugehörigen Ester mit niedrigem Alkylrest dargestellt. Diese Ester, vor allem die Acetate, besitzen holzige, erdige sowie grüne Geruchsnoten mit relativ geringer Intensität. Es ist anzunehmen, daß Spuren der zugehörigen geruchsintensiven Alkohole den Eigengeruch der Ester übertönen.

Die folgenden Beispiele erläutern die Erfindung.

Herstellungsbeispiel 1

1,5-Dimethyl-8-ethinyl-bicyclo[3.2.1]octan-8-ol (5)

Zu einer Suspension von 79,6 g KOH (pulverisiert) in 17,3 ml n-Butanol und 230 ml Benzol wurden bei 10°C 21,6 ml N,N-Dimethylformamid zugetropft. Sodann wurde etwa 1 Std. unter kräftigem Rühren Acetylengas (mit konz. $H_2SO_4$ gewaschen) eingeleitet. Unter fortwährendem Einleiten von Acetylen wurden 76 g (0,5 m) 4 innerhalb von 30 min zugetropft. Nach 30 min Rühren bei 10°C wurde mit ca. 100 ml Wasser zerlegt und aufgearbeitet. Aus dem Rohprodukt (ca. 88 g) wurden durch Destillation über eine Drehbandkolonne 64 g (72%) 5 erhalten; Kp. (1 mm): 60°C. Das Produkt erstarrte zu einer farblosen kristallinen Masse; Schmp. 33—35°C. — IR: 3500 (O—H); 3300, 2100 $cm^{-1}$ (—C≡C—H). — NMR ($CCl_4$): $\delta$=1,05 s (1-, 5-$CH_3$), 1,5, br. s (6-, 7-$CH_2$), 2,42 ppm, s (—C≡C—H).

$C_{12}H_{18}O$ (M = 178,26)

Herstellungsbeispiel 2

1,5-Dimethyl-8-vinyl-bicyclo[3.2.1]octan-8-ol (6)

Eine Lösung von 53,4 g (0,3 m) 5 in 150 ml Benzin wurde mit 500 mg Lindlar-Katalysator versetzt und in einer Wasserstoff-Atmosphäre geschüttelt (Normalbedingungen). Nach Aufnahme von 1 Äquivalent $H_2$ (6,72 l) wurde filtriert, eingeengt und destilliert. Man erhielt 46 g (84%) 6 als farbloses Öl vom Kp. (1 mm) 70°C. IR: 3500/3600 (O—H), 3080, 1635, 990, 910 $cm^{-1}$ (—CH=$CH_2$). — NMR ($CCl_4$): $\delta$=0,75, s (1-, 5-$CH_3$), 1,50/1,53, br, s (6-, 7-$CH_2$), 5,0—6,2 ppm (—CH=$CH_2$).

$C_{12}H_{20}O$ (M = 180,28)

Herstellungsbeispiel 3

1,5-Dimethyl-8-ethyl-bicyclo[3.2.1]octan-8-ol (7)

Eine Lösung von 178 g (1 m) 5 in 1000 ml dest. Methanol wurde mit 1 g Raney-Nickel versetzt und in einem Autoklaven hydriert (20 bar/20—30°C). Nach ca. 4 Std. war die Wasserstoff-Aufnahme beendet (2 Äquivalent $H_2$). Das Reaktionsgemisch wurde dem Autoklaven entnommen, filtriert, eingeengt und destilliert. Erhalten wurden 155 g (85%) 7 als farbloses Öl; Kp. (1 mm) 68°C. — IR: 3500/3600 $cm^{-1}$ (O—H). — NMR ($CCl_4$): $\delta$=0,88, s (1-, 5-$CH_2$), 0,97, t, J=7Hz (—$CH_2$—$CH_3$), 1,45 ppm, br. s (6-, 7-$CH_2$). — MS: m/e (%) = 153 (100, $M^+$—29), 135 (56), 125 (20), 123 (20), 107 (71), 99 (20), 95 (45), 69 (46).

$C_{12}H_{22}O$ (M = 182,30)

Herstellungsbeispiel 4

1,5,8-Trimethyl-bicyclo[3.2.1]octan-8-ol (8)

Zu einer Lösung von 45,8 g (0,3 m) 1,5-Dimethyl-bicyclo[3.2.1]octan-8-on in 150 ml trockenem Diethylether wurden bei Siedetemperatur unter Rühren 250 ml (178 g) einer 5%igen Lösung von Methyllithium (0,4 m) in Diethylether hinzugetropft. Nach 1 Std. Rühren bei Siedetemperatur wurden ca. 50 ml Wasser zugetropft. Extraktion mit Diethylether, Trocknen der vereinigten organischen Phasen über $Na_2SO_4$, Einengen i. Vac. und Destillation ergaben 37 g (73%) 8 als farbloses Öl; Kp. (1 mm) 74°C. — IR (als Ölfilm): 3500 $cm^{-1}$ (O—H). — NMR ($CCl_4$): $\delta$=0,87, s, 6H (1,5-$CH_3$), 1,05, s, 3H (8-$CH_3$), 1,5 ppm; br. s., 4H (6,7-$CH_2$). — MS: m/e (%) = 168 (33, $M^+$), 153 (28), 150 (27), 135 (46), 125 (30), 124 (18), 123 (31), 122 (20), 121 (42), 111 (11), 110 (10), 109 (16), 108 (19), 107 (85), . . ., 43 (100).

$C_{11}H_{20}O$ (M = 168,27)

## Herstellungsbeispiel 5

1,5-Dimethyl-8-ethyl-bicyclo[3.2.1]octan-8-ol (7)

Eine Lösung von 54,5 g (0,5 m) Ethylbromid in 150 ml trockenem Diethylether wurden unter Rühren einer Aufschlämmung von 12 g (0,5 m) Magnesium-Gries in 50 ml trockenem Diethylether derart zugetropft, daß die Mischung gelinde siedete. Der Lösung des Grignard-Reagenzes wurden 53,2 g (0,35 m) 1,5-Dimethylbicyclo[3.2.1]octan-8-on, gelöst in 70 ml trockenem Diethylether, innerhalb von 30 min zugetropft. Nach 2 Std. Rühren bei Siedetemperatur ließ man auf Raumtemperatur abkühlen, setzte 100 ml gesättigte $NH_4Cl$-Lösung hinzu, extrahierte mehrfach mit Ether, wusch die vereinigten organischen Phasen mit gesättigter $NaHCO_3$-Lösung, trocknete über $Na_2SO_4$ und destillierte i. Vac. das Lösungsmittel ab. Destillation des Rückstands ergab 54 g Produkt, das aus 40% 2 und 60% 7 bestand. Durch erneute Feindestillation wurden 18,5 g 7 erhalten; Kp. (1 mm): 68°C. — IR: 3500/3600 $cm^{-1}$ (O—H). — NMR ($CCl_4$): $\delta$=0,88, s (1-, 5-$CH_2$), 0,97, t, J=7Hz (—$CH_2$—$CH_3$), 1,45 ppm, br. s (6-, 7-$CH_2$). — MS: m/e (%)=153 (100, $M^+$—29), 135 (56), 125 (20), 123 (20), 107 (71), 99 (20), 95 (45), 69 (46).

$C_{12}H_{22}O$ (M=182,30)

## Herstellungsbeispiel 6

1,5-Dimethyl-8-isopropyl-bicyclo[3.2.1]octan-8-ol (10)

In 800 ml absol. Ether wurden 10,5 g (1,5 m) Lithium (feingeschnitten) und 76 g (0,5 m) Dimethylbicyclo[3.2.1]octan-8-on (4) vorgelegt. Bei —10°C ließ man innerhalb von 4 Std. eine Lösung von 92,3 g (0,75 m) Isopropylbromid in 300 ml absol. Ether zutropfen. Nach 1 Std. Rühren bei 40°C wurde hydrolysiert und aufgearbeitet. Aus dem Rohprodukt wurden durch Destillation über eine Drehbandkolonne 46 g (47%) 10 vom Kp. (1 mm) 76°C erhalten. IR: 3500/3600 $cm^{-1}$ (O—H). — NMR ($CCl_4$): $\delta$=0,91, s (1-, 5-$CH_3$), 1,15, d, J=7 Hz ($\underline{CH_3}$—CH—$\underline{CH_3}$), 1,55 ppm, br. s (6-, 7-$CH_2$).

$C_{13}H_{24}O$ (M=196,32)

## Beispiel 7

(Darstellung und spektroskopische Daten von 9, 11, 12, 13 und 17)

1,5-Dimethyl-8-propyl-bicyclo[3.2.1]octan-8-ol (9)

Darstellung analog Beispiel 5. Kp. (1 mm) 77°C. — IR: 3500/3600 $cm^{-1}$ (O—H). — NMR ($CCl_4$): $\delta$ = 0,90, s (1-, 5-$CH_3$) 0,93, br. t (—$CH_2$—$CH_3$), 1,48 ppm, br. s (6-, 7-$CH_2$).

$C_{13}H_{24}O$ (M=196,32)

8-Butyl-1,5-dimethyl-bicyclo[3.2.1]octan-8-ol (11)

Darstellung analog Beispiel 4. Kp. (2 mm) 94°C. — IR: 3500/3600 $cm^{-1}$ (O—H). — NMR ($CCl_4$): $\delta$ = 0,90, s (1-, 5-$CH_3$) 0,92, br. t (—$CH_2$—$CH_3$), 1,48 ppm, br. s (6-, 7-$CH_2$).

$C_{14}H_{26}O$ (M=210,35)

1,5-Dimethyl-8-2'-methyl-propyl-bicyclo[3.2.1]octan-8-ol (12)

Darstellung analog Beispiel 5. Kp. (1 mm) 92°C. — IR: 3500/3600 $cm^{-1}$ (O—H). — NMR ($CCl_4$): $\delta$ = 0,90, s (1-, 5-$CH_3$) 1,12, d, J=7 Hz (2'—$CH_3$), 1,58 ppm, br. s (6-, 7-$CH_2$).

$C_{14}H_{26}O$ (M=210,35)

1,5-Dimethyl-8-3'-methyl-propyl-bicyclo[3.2.1]octan-8-ol (13)

Darstellung analog Beispiel 5. Kp (1 mm) 80°C. — IR: 3600 cm $^{-1}$ (O—H). — NMR ($CCl_4$): $\delta$ = 0,90, s (1-, 5-$CH_3$) 0,98, d, J = 7Hz (3'—$CH_3$), 1,46 ppm, br. s (6-, 7-$CH_2$).

$C_{15}H_{28}O$ (M = 224,37)

1,5-Dimethyl-8-cyclopentyl-bicyclo[3.2.1]octan-8-ol (17)

Darstellung analog Beispiel 5. Kp. (1 mm) 112°C. — IR: 3500/3600 cm$^{-1}$ (O—H). — NMR ($CCl_4$): $\delta$ = 0,90, s (1-, 5-$CH_3$), 1,51 ppm, s (6-, 7-$CH_2$).

$C_{15}H_{28}O$ (M = 224,37)

Anwendungsbeispiel 8

Parfümöl »Eau de Cologne masculin«

| | |
|---|---|
| 300 g | Bergamottöl Reggio |
| 50 g | Petitgrainöl Paraguay |
| 200 g | Orangenöl Florida |
| 100 g | Citronenöl Messina |
| 10 g | Muskatnußöl |
| 60 g | Rosmarinöl spanisch |
| 50 g | Litsea Cubebaöl |
| 40 g | 3-Oxabicyclo[10.3.0]pentadec-6-en |
| 40 g | Calarenepoxid |
| 20 g | Corianderöl |
| 20 g | Cumarin |
| 15 g | Phenyläthylalkohol |
| 15 g | Thymol |
| 5 g | Cedernblätteröl, Virginia |
| 20 g | Dimethyltetrahydrobenzaldehyd 10% i. DPG |
| 50 g | Lavendelöl französisch |
| 995 g | |

Das angeführte Parfümöl ist ein Handelsprodukt, wirkt aber etwas unharmonisch; es treten krautige, fettig-süße Aspekte hervor. Durch Zugeben von nur 0,5 g einer 0,1%igen Lösung des 8-Ethyl-1,5-dimethyl-bicyclo[3.2.1]octan-8-ol (7) entsteht eine harmonische Komposition mit natürlicher Ausstrahlung. Die Agrumen-Aspekte werden betont und der Duftakkord wirkt mehr frisch und elegant.

Anwendungsbeispiel 9

Parfümöl »Fougère«

| | |
|---|---|
| 333,0 g | Lavandinoel Abrialis |
| 246,0 g | p-tert.-Butylcyclohexylacetat |
| 145,0 g | Cedrol-Fraktion (50%ig) |
| 45,0 g | Linalylacetat |
| 44,0 g | Dihydromyrcenol |
| 44,0 g | Eichenmoos-Extrakt |
| 29,0 g | Linalool |
| 22,0 g | Cumarin |
| 22,0 g | 1-Octen-3-ol, 1%ig |
| 22,0 g | Lavendel-Absolue |
| 14,0 g | Ambra-Tinktur, 1%ig in DPG |
| 9,0 g | Methylhexylketon, 10%ig in DPG |
| 7,0 g | Ethylenbrassylat |
| 4,0 g | $\alpha$-Jonon |
| 986,0 g | |

Dieses Parfümöl ist ein Fougère-Komplex mit etwas schwersüßlichen Aspekten. Nach Zugabe von 0,5 g einer 0,1%igen Lösung des 8-Ethyl-1,5-dimethyl-bicyclo-[3.2.1]octan-8-ols (in Ethanol) ist festzustellen, daß die schweren süßen Geruchsnoten zurückgedrängt werden zugunsten einer deutlich verstärkten Ausstrahlung unter Betonung frischer, holziger Aspekte mit Anklängen von Patchouly und Ambra.

Die Verbindung I kann zweckmäßig wie folgt angewandt werden:

a) als Riechstoff (in reiner Form)
b) als Bestandteil von Parfümölen oder Flavour-Mischungen, wobei übliche Konzentrationen 0,00001—30%, vorzugsweise 0,001—10% sind.

**Patentansprüche**

1. C-8-substituierte 1,5-Dimethyl-bicyclo[3.2.1]octan-8-ole nach allgemeiner Formel I

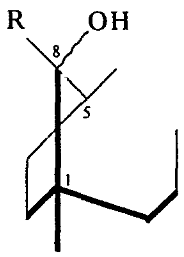

I

bzw.

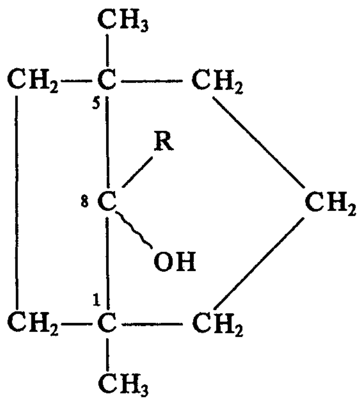

worin R eine niedere offenkettige oder zyklische Alkyl-, Alkenyl- oder Alkinylgruppe mit bis zu 6, vorzugsweise bis zu 5 Kohlenstoffatomen darstellt und die geschlängelte Linie am C-8-Atom epimere Formen bedeutet.

2. Verbindungen nach Anspruch 1, worin R eine geradkettige Alkylgruppe bedeutet.

3. Verbindungen nach Anspruch 1, worin R eine einfach verzweigt-kettige Alkylgruppe, insbesondere iso-Propyl, sek.-Butyl oder iso-Amyl bedeutet.

4. Verbindungen nach Anspruch 1, worin R eine Alkenylgruppe, insbesondere Vinyl, Allyl oder Dimethylallyl bedeutet.

5. Verbindungen nach Anspruch 1, worin R die Alkinylgruppe Äthinyl oder Propinyl bedeutet.

6. Verwendung der Verbindungen nach den Ansprüchen 1 bis 5 als Riechstoffe und Bestandteil von Parfümkompositionen für kosmetische und technische Parfümierungen.

7. Verwendung der Verbindungen nach den Ansprüchen 1 bis 6 in ätherischen Ölen und Aromen.

8. Verfahren zur Darstellung von Verbindungen nach den Ansprüchen 1 bis 6, dadurch gekennzeichnet, daß man 1,5-Dimethyl-bicyclo[3.2.1]octan-8-on in an sich bekannter Weise mit einer metall-organischen Verbindung umsetzt, die den Rest R oder einen Rest, der durch Hydrierung in R überführt wird, beinhaltet, anschließend, ggf. nach teilweiser oder vollständiger Hydrierung in an sich bekannter Weise, hydrolysiert und in an sich bekannter Weise isoliert.

9. Verfahren nach Anspruch 8, dadurch gekennzeichnet, daß die metall-organische Verbindung aus einem Alkalimetall und einer organischen Verbindung aufgebaut und in polaren aprotischen Solventien verwendet wird.

10. Verfahren nach Anspruch 8, dadurch gekennzeichnet, daß als Solventien Dimethylformamid, bzw. Diethylether oder Tetrahydrofuran verwendet wird.

**Claims**

1. C-8-substituted 1,5-dimethyl-bicyclo[3.2.1]octan-8-ols according to the general formula I

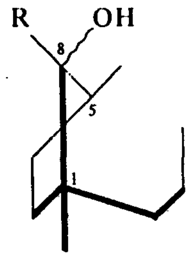

I

or

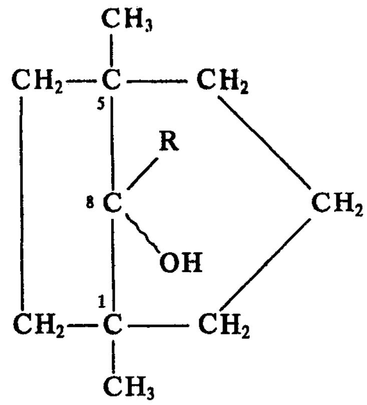

wherein R represents a lower open-chain or cyclic alkyl, alkenyl or alkynyl group having up to 6, preferably up to 5, carbon atoms and the wavy line on the C-8-atom denotes epimeric forms.

2. Compounds according to Claim 1, wherein R denotes a straight-chain alkyl group.

3. Compounds according to Claim 1, wherein R denotes an alkyl group with a chain having a single branch, in particular iso-propyl, sec.-butyl or iso-amyl.

4. Compounds according to Claim 1, wherein R denotes an alkenyl group, in particular vinyl, allyl or dimethylallyl.

5. Compounds according to Claim 1, wherein R denotes ethynyl or propynyl as the alkynyl group.

6. Use of the compounds according to Claims 1 to 5 as fragrances and as a constituent of perfume compositions for cosmetic and industrial perfuming.

7. Use of the compounds according to Claims 1 to 6 in essential oils and aromas.

8. Process for the preparation of compounds according to Claims 1 to 6, characterised in that 1,5-dimethylbicyclo[3.2.1]octan-8-one is reacted, in a manner known in itself, with a metal-organic compound which contains the radical R or a radical which is converted to R by hydrogenation, and the product is then, if appropriate after partial or complete hydrogenation in a manner known in itself, hydrolysed and isolated in a manner known in itself.

9. Process according to Claim 8, characterised in that the metal-organic compound is built up from an alkali metal and an organic compound and is used in polar aprotic solvents.

10. Process according to Claim 8, characterised in that dimethylformamide and/or diethyl ether or tetrahydrofuran are used as the solvents.

**Revendications**

1. 1,5-diméthyl-bicyclo[3.2.1]octanols-8 substitués en C-8 selon la formule générale I

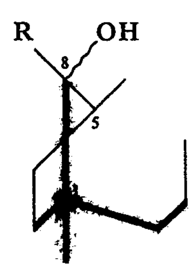

I

ou

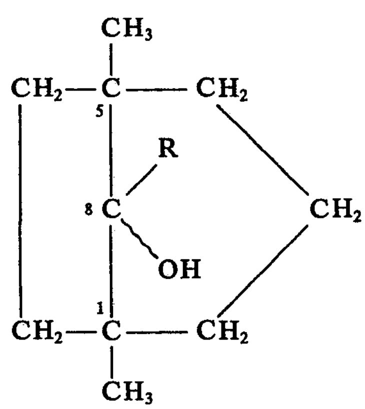

où R est un groupe alkyle, alcényle ou alcinyle inférieur, à chaîne ouverte ou cyclique, ayant jusqu'à 6 atomes de carbone, de préférence jusqu'à 5, le trait ondulé sur l'atome de C-8 représentant des formes épimères.

2. Composés selon la revendication 1, où R est un groupe alkyle à chaîne droite.

3. Composés selon la revendication 1, où R est un groupe alkyle à chaîne une fois ramifiée, en particulier les groupes isopropyle, sec-butyle ou isoamyle.

4. Composés selon la revendication 1, où R est un groupe alcényle, en particulier un groupe vinyle, allyle ou diméthylallyle.

5. Composés selon la revendication 1, où R est le groupe alcinyle, éthinyle ou propinyle.

6. Utilisation des composés selon les revendications 1 à 5 en tant que matières odorantes et constituants de compositions de parfum pour parfumages cosmétiques et techniques.

7. Utilisation des composés selon les revendications 1 à 6 dans des huiles essentielles et arômes.

8. Procédé pour la préparation de composés selon les revendications 1 à 6, caractérisé en ce qu'on fait réagir de la 1,5-diméthyl-bicyclo[3.2.1]octanone-8, d'une manière connue en soi, sur un composé organo-métallique, qui contient le radical R ou un radical qui est transformé en R par hydrogénation, puis qu'on l'hydrolyse, éventuellement après hydrogénation partielle ou totale d'une manière connue en soi, et qu'on l'isole d'une manière connue en soi.

9. Procédé selon la revendication 8, caractérisé en ce que le composé organo-métallique est constitué d'un métal alcalin et d'un composé organique, et est utilisé dans des solvants aprotiques polaires.

10. Procédé selon la revendication 8, caractérisé en ce qu'on utilise en tant que solvants le diméthylformamide, ou bien l'oxyde de diéthyle ou le tétrahydrofuranne.